(19) Europäisches Patentamt
European Patent Office
Office européen des brevets

(11) **EP 3 452 843 B1**

(12) **EUROPÄISCHE PATENTSCHRIFT**

(45) Veröffentlichungstag und Bekanntmachung des Hinweises auf die Patenterteilung:
**01.07.2020 Patentblatt 2020/27**

(21) Anmeldenummer: **17730361.7**

(22) Anmeldetag: **03.05.2017**

(51) Int Cl.:
**G01R 33/28** *(2006.01)*   **A61B 17/34** *(2006.01)*
**A61B 90/13** *(2016.01)*

(86) Internationale Anmeldenummer:
**PCT/DE2017/100367**

(87) Internationale Veröffentlichungsnummer:
**WO 2017/194051 (16.11.2017 Gazette 2017/46)**

(54) **OPERATIVES ASSISTENZSYSTEM FÜR EINEN MAGNETRESONANZTOMOGRAPHEN**

OPERATIVE ASSISTANCE SYSTEM FOR A MAGNETIC RESONANCE TOMOGRAPH

SYSTÈME D'ASSISTANCE OPÉRATIONNEL POUR TOMOGRAPHE À RÉSONANCE MAGNÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.05.2016 DE 102016005436**
**07.05.2016 DE 102016005437**

(43) Veröffentlichungstag der Anmeldung:
**13.03.2019 Patentblatt 2019/11**

(73) Patentinhaber: **Otto-von-Guericke-Universität Magdeburg**
**39106 Magdeburg (DE)**

(72) Erfinder:
• **PANNICKE, Enrico**
**39114 Magdeburg (DE)**

• **OPFERMANN, Klemens**
**39108 Magdeburg (DE)**

(74) Vertreter: **Gramm, Lins & Partner**
**Patent- und Rechtsanwälte PartGmbB**
**Freundallee 13a**
**30173 Hannover (DE)**

(56) Entgegenhaltungen:
**DE-A1-102005 046 077**

• **SCHOENENBERGER A W ET AL: "Punktionen an einem offenen, interventionellen MR-Gerät Veranschaulichung anhand eines Fallbeispiels", RADIOLOGE, DER, SPRINGER, DE, Bd. 36, Nr. 9, 19. Februar 2014 (2014-02-19), Seiten 705-708, XP035304069, ISSN: 0033-832X, DOI: 10.1007/S001170050131 [gefunden am 2014-02-19]**

**Beschreibung**

Die Erfindung betrifft ein operatives Assistenzsystem für einen Magnetresonanztomographen zur Anzeige eines Eintrittspunktes für ein Instrument auf der Oberfläche eines Patienten.

Minimal-invasive Behandlungsmethoden versuchen die Belastung für den Patienten unter anderem dadurch zu reduzieren, dass der Patient möglichst wenig verletzt wird. Dadurch entfällt aber auch die direkte visuelle Kontrolle während einer Behandlung. Deshalb ist es nötig eine solche Behandlung durch ein bildgebendes Verfahren zu begleiten. Die Magnetresonanztomographie ist hierfür besonders gut geeignet, da sie einen guten Weichteilkontrast liefert und die darzustellenden Schichten beliebig orientiert werden können. Grundlage für die Bildgenerierung ist der Wasserstoff im menschlichen Körper. Stoffe, die keinen Wasserstoff in geeigneter Form enthalten, können mit dieser Methode nicht dargestellt werden. Dies betrifft vor allem die Instrumente, die während der Behandlung nur durch ihre Wirkung im Patienten visualisiert werden können, üblicherweise die Verschiebung von Gewebe.

Für einen optimalen Behandlungsverlauf ist es auch notwendig, dass ein bestimmter Eintrittspunkt für das Instrument gewählt wird. Dieser kann von einer Vielzahl von Kriterien abhängen und wird vorher vom Arzt anhand diagnostischer Bilddaten geplant. Den vorher geplanten Eintrittspunkt auf dem Patienten zu finden erfordert ein hohes Maß an Vorstellungsvermögen seitens des Arztes. Dieser muss sich anhand der zweidimensionalen Schichtbilder des Inneren, auf der Oberfläche des Patienten orientieren. Es wäre also wünschenswert, den Arzt in dieser Aufgabe durch die Bereitstellung einer geeigneten Vorrichtung zu unterstützen, damit die Einstichstelle schneller lokalisiert werden kann.

In der Vergangenheit wurden verschiedene Methoden vorgestellt, um die Einstichstelle für ein Instrument auf dem Patienten zu finden.

Fingertip-Methode

Bei dieser Methode wird kontinuierlich die Ebene mit der Einstichstelle aufgenommen und der Arzt fährt mit dem Finger über den Patienten. Dabei wird dieser von einer MTA im Scannervorraum unterstützt, die die Bildgebung kontrolliert. Vorteil dieser Methode ist ihre simple und effiziente Umsetzung. Es sind keine zusätzlichen Werkzeuge zur Durchführung nötig. Jedoch benötigt der Arzt viel Erfahrung sich in den beliebigen Schichtorientierungen zu orientieren. Außerdem hängt die Effizienz der Methode stark vom Zusammenspiel der MTA mit dem Arzt ab. Kurzfristige Veränderungen im Team können hierbei beträchtliche Effizienzverluste verursachen.

Trackingsysteme

Trackingsysteme können verwendet werden, um ein Instrument unabhängig von der Bildgebung zu verfolgen. Diese können dann als "unabhängige" Kontrollinstanz wichtige Aufgaben übernehmen. Aktuelle und Zielkoordinate können problemlos miteinander abgeglichen werden, jedoch ist eine dem Arzt zugängliche Visualisierung nur schwer möglich. Auch ist ein solches Trackingsystem nicht überall verfügbar und kann immer nur ein kleines Volumen überwachen. Außerhalb dessen kann das Instrument nicht verfolgt werden und somit keine Korrektur geliefert werden, wie weit dieses von der Einstichstelle entfernt ist.

Marker

Instrumente können bildbasiert überwacht werden, indem deren Eigenschaften im Bild manipuliert werden. Diese Methode kann sehr effizient sein. Jedoch muss dafür dessen grobe Orientierung und Position bekannt sein, damit die aufzunehmenden Schichten entsprechend ausgerichtet werden können.

Planungssoftware

Anhand einer detaillierten Planung werden eindeutig bestimmbare Landmarken genutzt, um die Einstichstelle zu markieren. Dies kann z.B. dadurch geschehen, dass die Lage auf der z-Achse bekannt ist und die entsprechende Bogenlänge auf dem Patienten abgemessen wird. Nachteil dieser Methode ist, dass das Behandlungsszenario nicht von der Planung abweichen darf.

DE 102011006650 A1 offenbart eine Vorrichtung und ein Verfahren zur Behandlung einer Prostata in einem Patienten, mit einem Bildgebungsgerät zur Erzeugung eines Bildes der Prostata, mit einer freihändig geführten, durch einen Eintrittspunkt am Damm des Patienten in diesen einbringbaren Behandlungsnadel, mit einem das Bildgebungsgerät und die Behandlungsnadel koppelnden Navigationssystem und mit einem Führungsmittel zur Anzeige einer zu einem der Zielorte führende Soll-Trajektorie für die Behandlungsnadel, wobei für mindestens zwei der Zielorte die jeweiligen Soll-Trajektorien so gewählt sind, dass sie durch den selben Eintrittspunkt führen.

Bekannt aus DE20000107 U1 ist ein Magnetresonanztomograph (MRT) mit einem Laser-Leitsystem zur direkten Darstellung von Lokalisation und Zugangsweg körperinnerer Strukturen am Patienten mittels Laserstrahltechnik, wobei der Anwender mit einem daran angeschlossenen Computersystem und dessen Software die Zielstruktur lokalisieren, den Zugangsweg planen und den Laser computergesteuert mittels der diversen beweglichen Führungsschienen, Arme und Gelenke in die Position zur Projektion der geplanten Trajektorie bringen kann.

Aus DE 102005046077 A1 sind ein Magnetresonanztomograph und ein Verfahren zur Anzeige einer Einstichstelle oder Markierung am Körper eines Patienten für eine Biopsie oder einer Therapieanwendung bei einer MRT-Untersuchung bekannt. Die Markierung auf dem Patienten zum Auffinden der Einstichstelle erfolgt mittels Laser, wobei die Positioniereinrichtung, die vor oder in dem Thomographen angeordnet ist, wenigstens einen Motor zum Verfahren des Lasers entlang einer Führung aufweist und wobei der Motor aus nicht-magnetischen Teilen besteht und derart ausgebildet ist, dass während des Motorbetriebes kein eigenes Magnetfeld erzeugt wird.

Die bekannten Verfahren weisen jeweils Fremdantriebe zur Steuerung der Positioniereinrichtung auf.

Vor diesem Hintergrund wäre es wünschenswert, ein operatives Assistenzsystem für einen Magnetresonanztomographen zur Anzeige eines Eintrittspunktes für ein Instrument auf der Oberfläche eines Patienten zu haben, welches leicht handhabbar ist ohne dabei den Patienten drastisch zu beanspruchen bzw. gar zu verletzen.

Diese Aufgabe wird mit einer Vorrichtung nach Anspruch 1 oder 5 und einem Verfahren nach Anspruch 10 gelöst. Weitere vorteilhafte Ausführungsformen gehen aus den Unteransprüchen hervor.

Vorgeschlagen wird ein Operatives Assistenzsystem (1) für einen Magnetresonanztomographen (I) zur Anzeige eines Eintrittspunktes (IX) für ein Instrument auf der Oberfläche eines Patienten (XII), zumindest umfassend:

- eine drehbar gelagerte Einrichtung (3), umfassend zumindest

- eine erste Halterung (IV), an der zumindest einer Leiterschleife (III) aufgenommen ist, und

- eine zweite Halterung (V) mit zumindest einem Leuchtmittel (II) zur Anzeige von vorgebbaren Koordinaten des Eintrittspunktes (IX),

- wobei die Einrichtung (3) dazu geeignet ist, benachbart zu einer Mantelfläche ($I_M$) des Magnetresonanztomographen (I) angeordnet zu werden,

- eine Lagerung (VI) zur drehbaren Lagerung der Einrichtung (3) und

- eine Steuereinheit (SE) zur Veränderung der Position des Leuchtmittels (II),

wobei die Steuereinheit (SE) elektrisch mit der Leiterschleife (III) und dem Leuchtmittel (II) verbunden ist und die erste Halterung (IV) derart mit der zweiten Halterung (V) mittels Kraftübertragung zusammenwirkt, dass die Position des Leuchtmittels (II) veränderbar ist, wobei das Magnetfeld des Magnetresonanztomographen (I) genutzt ist, um durch die bestromte Leiterschleife (III) die Position des Leuchtmittels (II) zu verändern.

Dieser Aufbau kann sehr kompakt ausgeführt werden und ermöglicht es dennoch, eine große Variabilität in der Ausrichtung des Leuchtmittels. Der Einsatz der Leiterschleifen hat auch den großen Vorteil, dass die Anordnung immer einen stabilen Arbeitspunkt findet.

Wird eine Leiterschleife oder Spule im inhomogenen Magnetfeld des MRT bestromt, so wirkt auf diese aufgrund der bewegten Ladungsträger eine vom Magnetfeld abhängige Kraft. Diese führt zu einer Änderung der Ausrichtung durch ein Drehmoment und zu einer Beschleunigung des Objektes. Für den ersten Effekt ist die Feldstärke maßgeblich, während der zweite Effekt vom Gradienten des Feldes abhängt. Vor dem MRT im Bereich des magnetischen Streufeldes ist also davon auszugehen, dass die Beschleunigungswirkung dominiert, während das Objekt im homogenen Feld im Inneren des MRT seine Ausrichtung ändert. Im inhomogenen Feld erfolgt die Beschleunigung entlang der positiven Feldänderung d.h. zur größer werdenden Feldstärke hin.

Neben einer elektrischen Verbindung oder Übertragung von Signalen zwischen der Steuereinheit (SE) der Leiterschleife (III) und dem Leuchtmittel (II) ist auch eine ist auch eine optische Übertragung der Signale möglich. In einer Ausführungsform der Erfindung ist vorgesehen, dass das Assistenzsystem (1) an einer Innenwand der Mantelfläche ($I_M$) des Magnetresonanztomographen (I) angeordnet ist.

Dort besteht der Vorteil, dass es während der Behandlung wenig stört und den behandelnden Arzt nicht in die Augen leuchten kann.

Vorteilhafterweise umfasst das Assistenzsystem (1) zwei Leiterschleifen (III), die senkrecht zueinander angeordnet sind.

Dies führt dazu, dass sich das resultierende Dipolmoment als Vektoraddition der Momente der Leiterschleifen ergibt d.h. es ist eine sehr einfache Regelung der Auslenkung über das Verhältnis der Ströme in beiden Leiterschleifen möglich.

In einer Ausführungsform der Erfindung ist das Assistenzsystem (1) dadurch gekennzeichnet, dass die Einrichtung (3) in einem Winkel φ drehbar auf der Lagerung (VI) gelagert ist.

Vorzugsweise befindet sich das operative Assistenzsystem im homogenen Magnetfeld des Magnetresonanztomographen (I). Die Platzierung erfolgt vorzugsweise auf der Innenseite der Tomographenröhre (I). Dazu werden mindestens zwei Leiterschleifen (III) auf einer dafür vorgesehene Halterung (IV) befestigt. Die Halterungen (IV) sind mittels der Lagerung (VI) so gelagert, dass diese eine Drehbewegung durchführen können. Die beiden Leiterschleifen (III) sind senkrecht bzw. orthogonal zueinander orientiert. Das Verhältnis der in den Leiterschleifen (III) fließenden Ströme (VII) bestimmt den resultierenden Winkel φ.

Vorteilhafterweise sind die drehbaren Leiterschleifen leichtgängig gelagert und können separat im Hinblick auf ihre Polarität und Stromstärke angesteuert werden.

Es ist auch denkbar eine Messvorrichtung zu integrieren, die die Auslenkung der Anordnung erfassen kann. Damit können Abweichungen hervorgerufen durch die mechanische Konstruktion in einer Regelung ausgeglichen werden.

Die Leuchtmittel (II) des Assistenzsystem (1) können dabei ausgebildet sein als:

- Diode und/oder

- Laser und/oder

- Spiegelsystem und/oder

- Glühlampe mit Fokussierungseinrichtung

Es sind auch andere Ausführungsformen denkbar, die aber alle mindestens aus einer Leiterschleife (III) bestehen. Weitere Spulen sind vorzugsweise unterschiedlich bestromt. Bei geeigneter Lagerung (VI) und Umlenkung durch ein Getriebe (X) kann ein Leuchtmittel (II) in einer Art und Weise ausgerichtet werden, dass ein Zielpunkt (IX) auf der Patientenoberfläche (XII) markiert wird.

Verfahrensgemäß kann das operative Assistenzsystem (1) zur Anzeige eines Eintrittspunktes (IX) für ein Instrument in einem Magnetresonanztomographen (I) genutzt werden.

Vorgeschlagen wird weiterhin ein Operatives Assistenzsystem (1) für einen Magnetresonanztomographen (I) zur Anzeige oder Markierung eines Eintrittspunktes (IX) für ein Instrument auf der Oberfläche eines Patienten (VIII), zumindest umfassend:

- eine beweglich gelagerte Einrichtung (3a, 3b, 3c), umfassend zumindest

- eine erste Halterung (IV), an der zumindest eine Leiterschleife (III) aufgenommen ist und

- eine zweite Halterung (V) mit zumindest einem Leuchtmittel (II) zur Markierung von vorgebbaren Koordinaten des Eintrittspunktes (IX),

- wobei die Einrichtung (3a, 3b, 3c) dazu geeignet ist, unmittelbar vor der Öffnung des Magnetresonanztomographen (I) angeordnet zu werden,

- eine Lagerung (VI) zur beweglichen Lagerung der Einrichtung (3) und

- eine Steuereinheit (SE) zur Veränderung der Position des Leuchtmittels (II),

wobei die Steuereinheit (SE) elektrisch mit der Leiterschleife (III) und dem Leuchtmittel (II) verbunden ist und die erste Halterung (IV) derart mit der zweiten Halterung (V) mittels Kraftübertragung zusammenwirkt, dass die Position des Leuchtmittels (II) veränderbar ist, wobei das Magnetfeld des Magnetresonanztomographen (I) genutzt ist, um durch die bestromte Leiterschleife (III) die Position des Leuchtmittels (II) zu verändern. Dies führt dazu, dass auf der Patientenoberfläche (XII) die Position der Markierung verändert werden kann. Der Arzt wird dadurch z.B. im Auffinden einer Einstichstelle unterstützt.

Die Erfindung beruht auf der Kraftwirkung stromdurchflossener Leiter in einem Magnetfeld. In einem inhomogenen Streufeld erfahren diese Leiter eine Beschleunigung entlang des Gradienten des Feldes. Durch entsprechende Planung und Lagerung der Leiterschleifen kann die Kraftwirkung zur Auslenkung anderer Vorrichtungen oder Einrichtungen verwendet werden.

Die beweglich gelagerte Einrichtung (3a, 3b, 3c) kann als Kreisring oder als gerade ausgebildete Einrichtung rotatorisch und/oder translatorisch im inhomogenen Magnetfeld außerhalb des Tomographen oder Magnetresonanztomographen (MRT), jedoch unmittelbar angrenzend an diesen geführt werden. Damit befindet sich das operative Assistenzsystem an sich außerhalb des homogenen Magnetfelds des Magnetresonanztomographen (I). Die Platzierung erfolgt vorzugsweise an der Außenseite der Tomographenröhre (XI). Neben einer elektrischen Verbindung oder Übertragung von Signalen zwischen der Steuereinheit (SE), der Leiterschleife (III) und dem Leuchtmittel (II) ist auch eine optische Übertragung der Signale möglich.

Eine Ausführungsform der Erfindung sieht vor, dass die erste Halterung (IV) in der Einrichtung (3a) zumindest bogenförmig ausgebildet ist und ein oder mehrere Leiterschleifen (III) mit Stromanschlüssen (VII) auf der ersten Halterung (IV) angeordnet sind, wobei die Leiterschleifen (III) separat mittels Strom ansteuerbar sind und im Streufeld des Magnetresonanztomographen (I) eine Kraft wirkt und die erste Halterung (IV) derart mit der zweiten Halterung (V) mittels Kraftübertragung zusammenwirkt, dass die Halterung (IV) um eine Längsachse L des Magnetresonanztomographen (I) rotierbar ist und die Koordinaten des Eintrittspunktes (IX) für das Instrument auf der Oberfläche des Patienten (VIII) markierbar sind.

Vorteilhafterweise ist das Leuchtmittel (II) über die Halterung (V) starr mit der ersten Halterung (IV) verbunden.

Weiterhin ist vorgesehen, dass die erste Halterung (IV) in der Einrichtung (3b) zumindest gerade ausgebildet ist und zumindest eine Leiterschleife (III) mit Stromanschlüssen (VII) auf der ersten Halterung (IV) angeordnet ist, wobei die Leiterschleife (III) mittels Strom ansteuerbar ist und eine Kraft wirkt und die erste Halterung (IV) derart mit der zweiten Halterung (V) mittels Kraftübertragung zusammenwirkt, dass die erste Halterung (IV) translatorisch oder linear in Pfeilrichtung T bewegbar ist, derart, dass die zweite Halterung (V) in Pfeilrichtung R rotierbar ist und die Koordinaten des Eintrittspunktes (IX) für das Instrument auf der Oberfläche des Patienten (VIII) markierbar sind. Ebenso kann diese Ausführungsform neben der geraden Ausbildung der ersten Halterung (IV) in der Einrichtung (3b) auch mit jeder anderen Geometrie ausgebildet sein.

Vorteilhafterweise ist die erste Halterung (IV) als eine Zahnstange ausgebildet.

In einer weiteren Ausführungsform der Erfindung ist vorgesehen, dass das Assistenzsystem einen Seilzug aufweist, der mit der ersten Halterung (IV) und der zweiten Halterung (V) mittels Kraftübertragung derart zusammenwirkt, dass die Ausrichtung der zweiten Halterung (V) in Pfeilrichtung T oder R veränderbar ist und mittels der Leuchtmittel (II) Koordinaten des Eintrittspunktes (IX) auf der Oberfläche eines Patienten (VIII) markierbar sind.

Die Leuchtmittel (II) des Assistenzsystems (1) können dabei ausgebildet sein als Diode und/oder Laser und/oder Spiegelsystem und/oder Glühlampe mit Fokussierungseinrichtung.

Es sind auch andere Ausführungsformen denkbar, die aber alle mindestens eine Spule oder Leiterschleife (III) umfassen. Weitere Spulen sind vorzugsweise unterschiedlich bestromt. Bei geeigneter Lagerung (VI) und Umlenkung der Kraft durch ein Getriebe (X) kann das Leuchtmittel II in einer Art und Weise ausgerichtet werden, dass der Zielpunkt (IX) auf der Patientenoberfläche (XII) markiert wird.

Verfahrensgemäß kann das operative Assistenzsystem (1) zur Anzeige eines Eintrittspunktes (IX) für ein Instrument in einem Magnetresonanztomographen (I) dazu genutzt werden, vorgegebene Koordinaten zu verarbeiten (z.B. Umrechnung in unterschiedliche Referenzsysteme) und die Einrichtung (3a, 3b, 3c) entsprechend zu verfahren.

Die Erfindung wird nachfolgend anhand von Figuren näher erläutert, ist jedoch hierauf nicht beschränkt.

Es zeigen:

Fig. 1a und 1b: schematisch ein operatives Assistenzsystem für einen Magnetresonanztomographen (MRT) und eine Vektoraddition der Dipolmomente,

Fig. 2: schematisch eine Einzelheit (A) eines Getriebes (X) des operativen Assistenzsystems aus Fig. 1 und

Fig. 3: schematisch eine Einzelheit (A') zur Auslenkung des Leuchtmittels II.

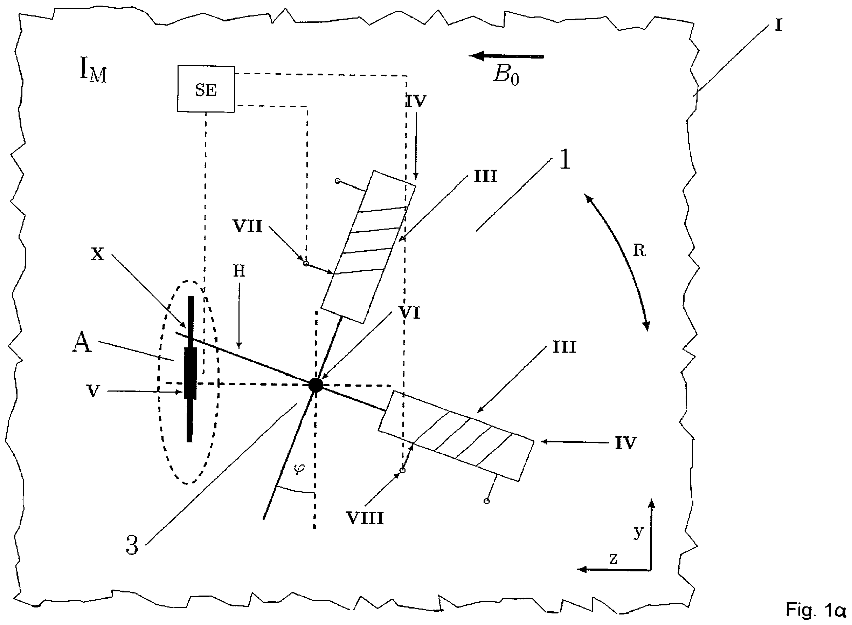

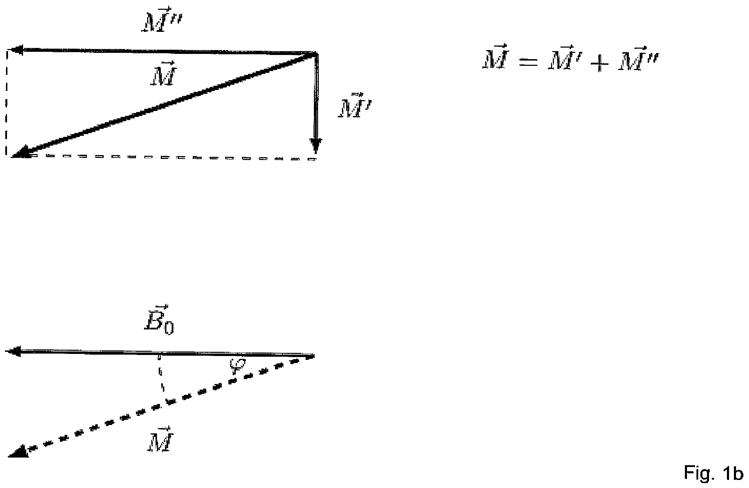

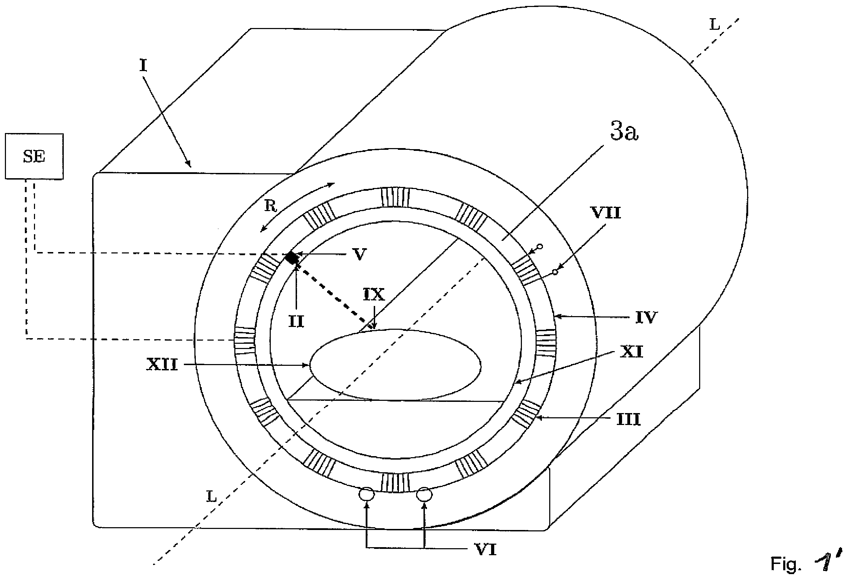

Fig. 1': schematisch ein operatives Assistenzsystem für einen Magnetresonanztomographen (MRT) und

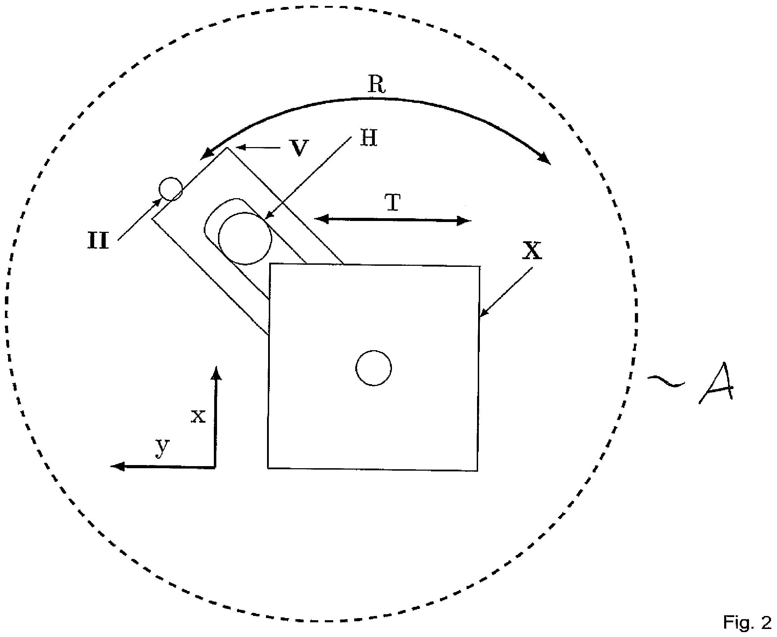

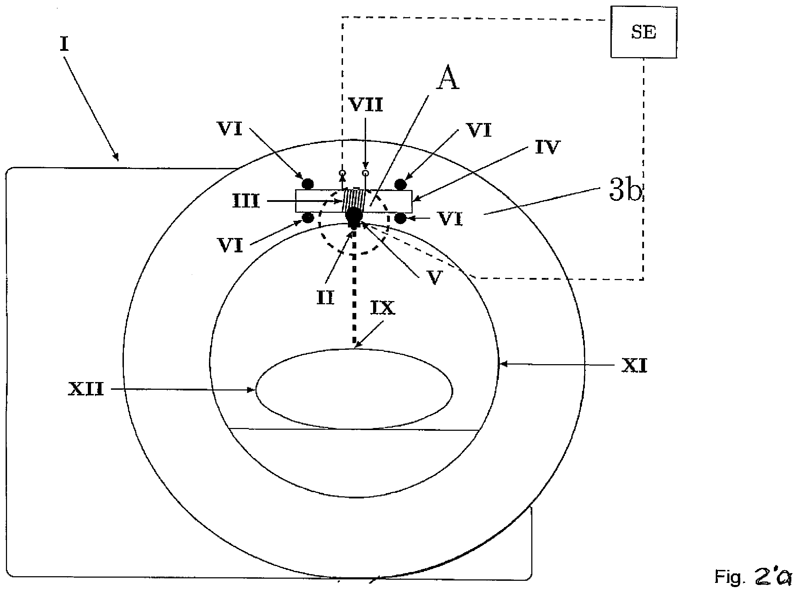

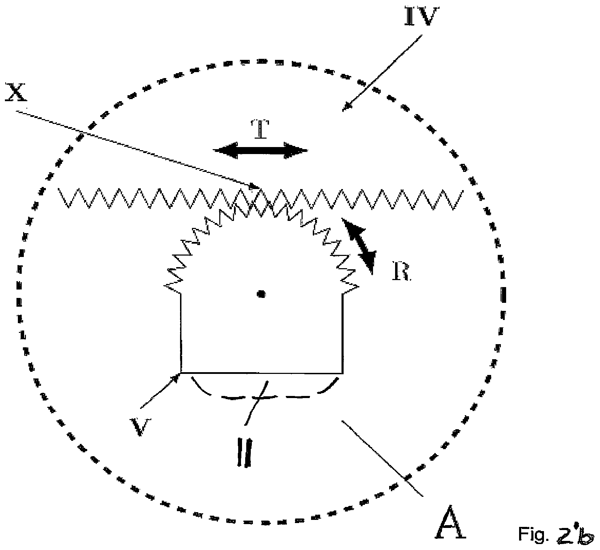

Fig. 2'a und 2'b: schematisch eine weitere Ausführung für ein operatives Assistenzsystem für einen Magnetresonanztomographen mit einer Einzelheit (A) eines Getriebes (X) des operativen Assistenzsystems und

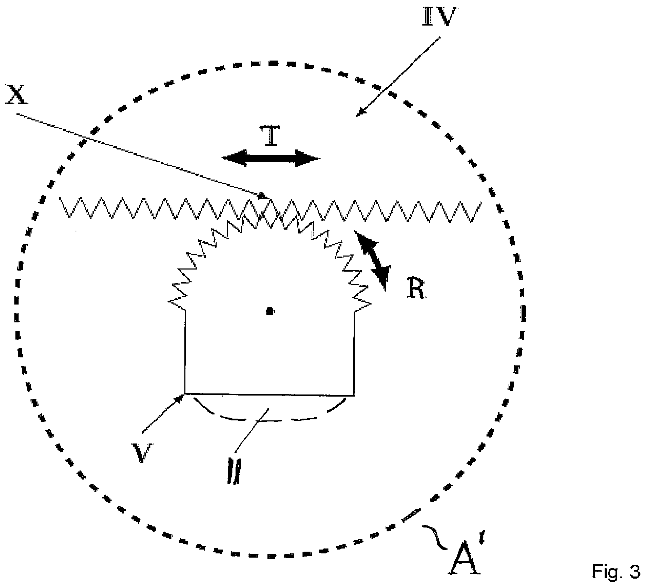

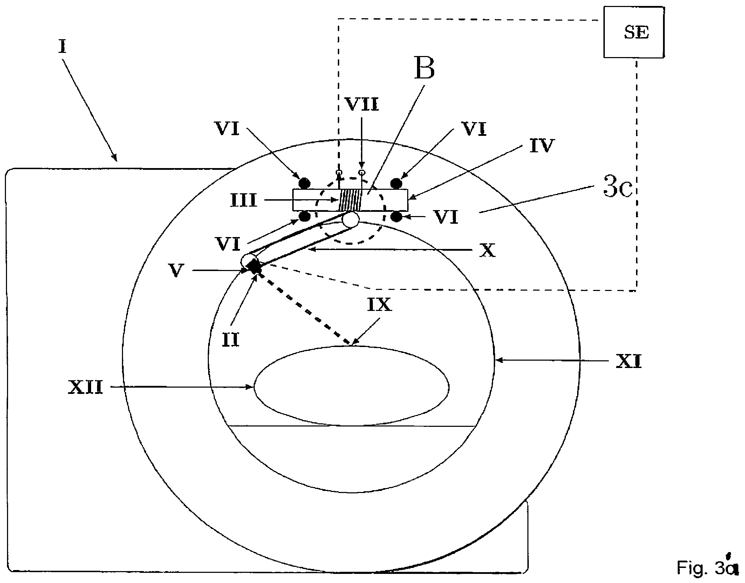

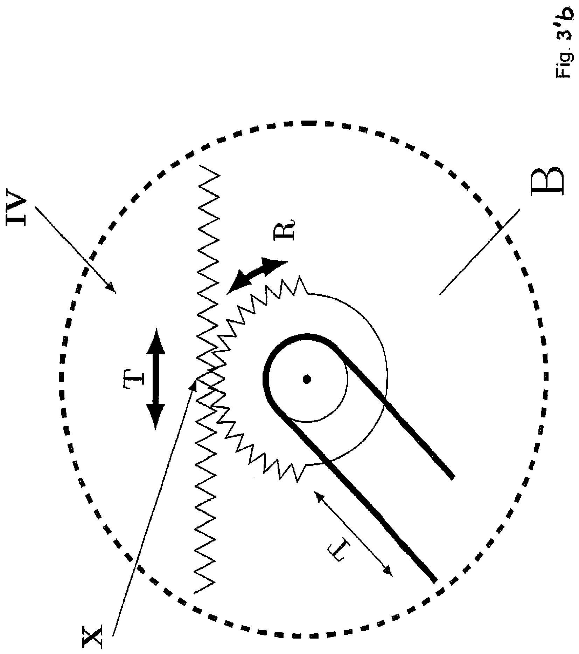

Fig. 3'a und 3'b: schematisch eine weitere Ausführung für ein operatives Assistenzsystem für einen Magnetresonanztomographen mit einer Einzelheit (B) eines Getriebes (X) des operativen Assistenzsystems.

In den Figuren 1a und 1b wird schematisch ein operatives Assistenzsystem für einen Magnetresonanztomographen zur Anzeige eines Eintrittspunktes (IX) für ein Instrument auf der Oberfläche eines Patienten (XII) gezeigt, wobei die Vektoraddition der Dipolmomente, die zur Ausrichtung der Anordnung des Positioniersystems bzw. der Positioniereinrichtung veranschaulicht ist.

Dabei ist eine drehbar gelagerte Einrichtung (3) vorgesehen, die eine erste Halterung (IV) zur Aufnahme von zumindest einer Leiterschleife (III) und eine zweite Halterung (V) zur Aufnahme von zumindest einem Leuchtmittel (II) gemäß der Einzelheiten (A) und (A') der Figuren 2 und 3 aufweist.

Das Leuchtmittel (II) dient dabei zur Anzeige von vorgebbaren Koordinaten des Eintrittspunktes (IX), wobei die Einrichtung (3) benachbart zu einer Mantelfläche ($I_M$) des Magnetresonanztomographen (I) angeordnet ist. Das Assistenzsystem weist eine Lagerung (VI) auf und eine Steuereinheit (SE) zur Veränderung der Position (Ausrichtung) des Leuchtmittels (II), wobei die Steuereinheit (SE) elektrisch mit der Leiterschleife (III) und dem Leuchtmittel (II) verbunden ist und die erste Halterung (IV) über zumindest eine Einrichtung in Form einer dritte Halterung (H) derart mit der zweiten Halterung (V) mittels Kraftübertragung zusammenwirkt, dass die Position des Leuchtmittels (II) veränderbar ist. Die Halterung (H) kann dabei als ein Hebel oder eine Kreisscheibe ausgebildet sein.

Das Assistenzsystem (1) ist an einer Innenwand der Mantelfläche (IM) des Magnetresonanztomographen (I) in der Koordinatenebene "y" und "z" angeordnet. Es umfasst beispielsweise zwei Leiterschleifen (III), die senkrecht zueinander angeordnet sind. Hierbei ist die Einrichtung (3) in einem Winkel φ drehbar auf der Lagerung (VI) gelagert.

Die Leiterschleife kann beispielsweise aus einem Kupferlackdraht mit einem Durchmesser von 0,15 mm gefertigt sein und ca. 100 Windungen aufweisen. Bei Verwendung von zwei Leiterschleifen sind diese vorzugsweise senkrecht bzw. orthogonal zueinander ausgerichtet. Die Leiterschleifen sind leichtgängig gelagert und können seitens ihrer Polarität und Stromstärke separat angesteuert werden, beispielsweise mit einem Strom von 100 mA. Das operative Assistenzsystem ist vorzugsweise aus MR-kompatiblen Materialien gefertigt.

Wird eine Leiterschleife oder Spule im homogenen Magnetfeld des MRT mittels fließender Ströme (VII, VIII) bestromt, so entsteht um diese Spule in Abhängigkeit vom fließenden Strom ein Magnetfeld. Dieses Magnetfeld tritt mit dem statischen Bo-Feld des MRT oder Tomographen in Wechselwirkung. Im homogenen Magnetfeld führt dies dazu, dass ein Drehmoment auf die Leiterschleife wirkt, das versucht deren magnetisches Dipolmoment parallel zum $B_0$-Feld auszurichten.

Werden zwei Leiterschleifen oder Spulen bestromt, so ist die resultierende Kraftwirkung über die Vektoraddition der beiden einzelnen Dipolmomente der Spulen wie folgt mathematisch bestimmbar:

$$\vec{M} = \vec{M}' + \vec{M}''$$

Die Positionierung eines Leuchtmittels kann somit über die unterschiedliche Bestromung der Spulen erfolgen. Hierdurch sind beliebige Winkel φ einstellbar. Indem das Magnetfeld des Magnetresonanztomographen durch bestromte Leiterschleifen genutzt wird, sind keine Fremdantriebe, wie beispielsweise Piezo- Antriebe oder pneumatische Antriebe nötig, um die Position des Leuchtmittels (II) zu verändern. Die erste Halterung (IV) ist in Kombination mit einem Getriebe (X) über die zweite Halterung (V) geeignet, das Leuchtmittel (II) in Rotationsrichtung R auszurichten.

Die Leuchtmittel (II) des Assistenzsystem (1) sind dabei als eine Diode und/oder ein Laser und/oder ein Spiegelsystem und/oder eine Glühlampe mit Fokussierungseinrichtung ausgebildet.

In Fig. 2 wird schematisch die Einzelheit (A) des Getriebes (X) des operativen Assistenzsystems aus Fig. 1 in der Koordinatenebene "y" und "x" gezeigt, wobei das Zusammenwirken des Getriebes (X) mit der dritten Halterung (H) beispielhaft und schematisch dargestellt ist. Ein Pfeil (T) kennzeichnet dabei die "ebene" Kraftwirkung.

Hierbei ist erkennbar, dass die Halterung (H) auf eine Art und Weise mit der Halterung (V) verbunden ist, dass die Auslenkung (R-Richtung) der Halterung (H), ebenfalls zu sehen in Fig. 1, zu einer Kraftwirkung auf (V) führt, d.h. zu einer Bewegung in T-Richtung. Durch die Ausführung des Getriebes (X) wird die Kraftwirkung in eine Rotation (R) umgesetzt, die dazu führt, dass sich das Leuchtmittel (II) auf einem Kreisbogen in R-Richtung verfahren lässt.

Eine Auslenkung des Leuchtmittels (II) kann beispielhaft auch über die Anordnung gemäß Einzelheit (A') in Fig. 3 erfolgen.

Die Lagerung (IV) ist auf eine Art und Weise mit der Halterung (H) verbunden, dass deren Rotation (R) zu einer Verschiebung in Richtung (T) führt. Das Getriebe (X) kann z.B. als Zahnstange ausgebildet sein und überträgt die Verschiebung (T) in eine Rotation (R) der Halterung (V), auf der vorzugsweise das Leuchtmittel (II) befestigt werden kann.

In Figur 1' wird schematisch ein operatives Assistenzsystem für einen Magnetresonanztomographen zur Anzeige eines Eintrittspunktes (IX) für ein Instrument auf der Oberfläche eines Patienten (XII) gezeigt.

Dabei ist eine drehbar gelagerte Einrichtung (3a) vorgesehen, die eine erste Halterung (IV) zur Aufnahme von zumindest einer Leiterschleife (III) und eine zweite Halterung (V) zur Aufnahme von zumindest einem Leuchtmittel (II) aufweist. Das Leuchtmittel (II) dient dabei zur Markierung bzw. Anzeige von vorgebbaren Koordinaten des Eintrittspunktes (IX), wobei die Einrichtung (3a) unmittelbar vor der Öffnung des Magnetresonanztomographen (I) angeordnet ist. Das Assistenzsystem weist eine Lagerung (VI) auf und eine Steuereinheit (SE) zur Veränderung der Position des Leuchtmittels bzw. Ausrichtung des Leuchtmittels (II), wobei die Steuereinheit (SE) elektrisch mit der Leiterschleife (III) und dem Leuchtmittel (II) verbunden ist und die erste Halterung (IV) derart mit der zweiten Halterung (V) mittels Kraftübertragung zusammenwirkt, dass die Position des Leuchtmittels (II) veränderbar ist.

Diese Anordnung befindet sich in unmittelbarer Nähe vor einem Magnetresonanztomographen I. Die Halterung IV für die Leiterschleifen III in Fig. V ist als Bogen ausgeführt, der mittels einer geeigneten Lagerung VI eine Rotationsbewegung um die Längsachse L des Magnetresonanztomographen I durchführen kann. Auf der Halterung IV sind eine Mehrzahl von Leiterschleifen III befestigt, die separat angesteuert werden können. Fließt durch eine Leiterschleife III ein Strom VII so wirkt auf diese im Streufeld des Tomographen I eine Kraft, die die Halterung IV rotieren lässt. Das Leuchtmittel II ist mit einer starren Halterung V mit dem Bogen verbunden. Durch eine Rotation des Bogens können verschiedene Zielpunkte IX auf dem Patienten XII markiert werden.

Die beweglich gelagerte Einrichtung (3a) kann als Kreisring ausgebildete sein und rotorisch im inhomogenen Magnetfeld außerhalb des Tomographen oder Magnetresonanztomographen (MRT), jedoch unmittelbar angrenzend an diesen geführt werden. Damit befindet sich das operative Assistenzsystem an sich außerhalb des homogenen Magnetfeldes des Magnetresonanztomographen (I). Die Platzierung erfolgt vorzugsweise an der Außenseite der Tomographenröhre (XI).

Die erste Halterung (IV) in der Einrichtung (3a) ist aber zumindest bogenförmig ausgebildet, wobei an ihr ein oder mehrere Leiterschleifen (III) mit Stromanschlüssen (VII) auf der ersten Halterung (IV) angeordnet sind, wobei die Leiterschleifen (III) separat mittels Strom ansteuerbar sind und im Streufeld des Magnetresonanztomographen (I) eine Kraft wirkt und die erste Halterung (IV) derart mit der zweiten Halterung (V) mittels Kraftübertragung zusammenwirkt, dass die Halterung (IV) um eine Längsachse L des Magnetresonanztomographen (I) rotierbar ist und die Koordinaten des Eintrittspunktes (IX) für das Instrument auf der Oberfläche des Patienten (VIII) markierbar sind.

Dabei ist das Leuchtmittel (II) über die Halterung (V) starr mit der ersten Halterung (IV) verbunden.

Die Leiterschleife (III) kann beispielsweise aus einem Kupferlackdraht mit einem Durchmesser von 0,15 mm gefertigt sein und ca. 100 Windungen aufweisen. Die Leiterschleifen können seitens ihrer Polarität und Stromstärke separat bestromt werden, beispielsweise mit einem Strom von 100 mA. Das operative Assistenzsystem ist vorzugsweise aus MR-kompatiblen Materialien gefertigt.

Die Leuchtmittel (II) des Assistenzsystem (1) sind dabei als eine Diode und/oder ein Laser und/oder ein Spiegelsystem und/oder eine Glühlampe mit Fokussierungseinrichtung ausgebildet.

In Fig. 2'a wird schematisch eine weitere Ausführung für ein operatives Assistenzsystem für einen Magnetresonanztomographen hinsichtlich der Halterung (IV) der Leiterschleife (II) und in Fig. 2'b schematisch eine Einzelheit A eines Getriebes (X) des operativen Assistenzsystems (1) gezeigt.

Hierbei ist die erste Halterung (IV) in der Einrichtung (3b) zumindest gerade ausgebildet und zumindest eine Leiterschleife (III) mit Stromanschlüssen (VII) auf der ersten Halterung (IV) angeordnet ist, wobei die Leiterschleife (III) mittels Strom ansteuerbar ist und eine Kraft wirkt und die erste Halterung (IV) derart mit der zweiten Halterung (V) mittels Kraftübertragung zusammenwirkt, dass die erste Halterung (IV) translatorisch (linear) in Pfeilrichtung T bewegbar ist, derart, dass die zweite Halterung (V) in Pfeilrichtung R rotierbar ist und die Koordinaten des Eintrittspunktes (IX) für das Instrument auf der Oberfläche des Patienten (VIII) markierbar sind. Ebenso kann diese Ausführungsform neben der geraden Ausbildung der ersten Halterung (IV) in der Einrichtung (3b) auch mit jeder anderen Geometrie ausgebildet sein.

Vorteilhafterweise ist die erste Halterung (IV) als eine Zahnstange ausgebildet.

In dieser Ausführung gemäß Fig. 2'a ist die Halterung (IV) als Linearführung ausgebildet. Wird durch die Leiterschleife (III) ein Strom (VII) geleitet, entsteht eine Kraft welche in Kombination mit der Lagerung, die Spulenhalterung linear führt. In Abhängigkeit der Polarität des Stromes (VII), kann damit eine translatorische Bewegung in T-Richtung nach links oder rechts erzeugt werden. Die lineare Bewegung wird durch das Getriebe (X), in Form der Zahnstange in eine Rotation der Halterung (V) des Leuchtmittels (II) überführt. Diese Rotation ermöglicht es, das Leuchtmittel (II) so auszurichten, dass verschiedene Zielpunkte (IX) auf dem Patienten (XII) markiert werden können, wie in Fig. 2'b dargestellt. Durch die Einzelheit (A) in Figur 2'b wird das Zusammenwirken eines Getriebe (X) mit der ersten Halterung (IV) und der zweiten Halterung (V) dargestellt.

Durch die Einzelheit (A) gemäß Fig. 2'b als Ausschnitt aus Figur 2'a wird das Zusammenwirken eines Getriebes (X) mit der ersten Halterung (IV) und der zweiten Halterung (V) dargestellt.

In den Fig. 3'a und 3'b wird schematisch eine weitere Ausführung für ein operatives Assistenzsystem für einen Magnetresonanztomographen mit einer Einzelheit (B) eines Getriebes (X) des operativen Assistenzsystems gezeigt.

In dieser Ausführungsform der Erfindung in Fig. 3'a ist vorgesehen, dass das Assistenzsystem einen Seilzug aufweist, der mit der ersten Halterung (IV) und der zweiten Halterung (V) mittels Kraftübertragung derart zusammenwirkt, dass die Ausrichtung der zweiten Halterung (V) in Pfeilrichtung T oder R veränderbar ist und mittels der Leuchtmittel (II) Koordinaten des Eintrittspunktes (IX) auf der Oberfläche eines Patienten (VIII) markierbar sind.

Dabei kann das Leuchtmittel (II) auch an einer anderen Stelle platziert werden.

Die Kraftübertragung zwischen der Halterung der Leiterschleife (IV) und der Halterung des Leuchtmittels (V), erfolgt hier durch ein Getriebe (X), das in den Fig. 3'a und 3'b als Seilzug ausgeführt ist, wobei in Fig. 3'b die Einzelheit (B) des Getriebes (X) dargestellt ist.

Es ist auch denkbar, die Leiterschleife (III) an einer anderen Stelle des Tomographen (I) zu platzieren. Es muss jedoch darauf geachtet werden, dass sich dadurch die Kraftwirkung ändert. Im Streufeld wird eine Beschleunigung und im homogenen Magnetfeld des Inneren eine Rotation des Objektes auftreten. Wichtig ist die richtige Wahl des Getriebes (X), dass für eine geeignete Kraftübertragung zwischen der Halterung (IV) der Leiterschleife (III) und der Halterung (V) des Leuchtmittels (II) sorgt.

Die beweglich gelagerte Einrichtung (3b, 3c) kann als gerade ausgebildete Einrichtung rotatorisch und/oder translatorisch im inhomogenen Magnetfeld außerhalb des Tomographen oder Magnetresonanztomographen (MRT), jedoch unmittelbar angrenzend an diesen geführt werden. Damit befindet sich das operative Assistenzsystem an sich außerhalb des homogenen Magnetfelds des Magnetresonanztomographen (I). Die Platzierung erfolgt vorzugsweise an der Außenseite der Tomographenröhre (XI).

Indem das Magnetfeld des Magnetresonanztomographen durch bestromte Leiterschleifen (III) genutzt wird, sind keine Fremdantriebe, wie beispielsweise Piezo-Antriebe oder pneumatische Antriebe nötig, um die Position des Leuchtmittels (II) zu verändern. Die erste Halterung (IV) ist in Kombination mit dem Getriebe (X) über die zweite Halterung (V) geeignet rotatorische bzw. translatorische Bewegungen auszuführen und das Leuchtmittel (II) in Rotationsrichtung R oder Translationsrichtung T auszurichten.

**Patentansprüche**

1. Operatives Assistenzsystem (1) für einen Magnetresonanztomographen (I) zur Anzeige eines Eintrittspunktes (IX) für ein Instrument auf der Oberfläche eines Patienten (VIII), zumindest umfassend:

    - eine drehbar gelagerte Einrichtung (3), umfassend zumindest

- eine erste Halterung (IV), an der zumindest eine Leiterschleife (III) aufgenommen ist, und

- eine zweite Halterung (V) mit zumindest einem Leuchtmittel (II) zur Anzeige von vorgebbaren Koordinaten des Eintrittspunktes (IX),
- wobei die Einrichtung (3) dazu geeignet ist, benachbart zu einer Mantelfläche ($I_M$) des Magnetresonanztomographen (I) angeordnet zu werden,
- eine Lagerung (VI) zur drehbaren Lagerung der Einrichtung (3) und
- eine Steuereinheit (SE) zur Veränderung der Position des Leuchtmittels (II),

wobei die Steuereinheit (SE) elektrisch mit der Leiterschleife (III) und dem Leuchtmittel (II) verbunden ist und die erste Halterung (IV) derart mit der zweiten Halterung (V) mittels Kraftübertragung zusammenwirkt, dass die Position des Leuchtmittels (II) veränderbar ist, wobei das Magnetfeld des Magnetresonanztomographen (I) genutzt ist, um durch die bestromte Leiterschleife (III) die Position des Leuchtmittels (II) zu verändern.

2. Assistenzsystem (1) nach Anspruch 1, **dadurch gekennzeichnet, dass** das Assistenzsystem (1) geeignet ist an einer Innenwand der Mantelfläche ($I_M$) des Magnetresonanztomographen (I) angeordnet zu werden.

3. Assistenzsystem (1) nach Anspruch 1 oder 2, umfassend zwei an der ersten Halterung (IV) angeordnete Leiterschleifen (III), die senkrecht zueinander angeordnet sind.

4. Assistenzsystem (1) nach einem der vorhergehenden Ansprüche, **dadurch gekennzeichnet, dass** die Einrichtung (3) in einem Winkel φ drehbar auf der Lagerung (VI) gelagert ist.

5. Operatives Assistenzsystem (1) für einen Magnetresonanztomographen (I) zur Anzeige eines Eintrittspunktes (IX) für ein Instrument auf der Oberfläche eines Patienten (VIII), zumindest umfassend:

- eine beweglich gelagerte Einrichtung (3a, 3b, 3c), umfassend zumindest

- eine erste Halterung (IV), an der zumindest eine Leiterschleife (III) aufgenommen ist, und

- eine zweite Halterung (V) mit zumindest einem Leuchtmittel (II) zur Markierung von vorgebbaren Koordinaten des Eintrittspunktes (IX),
- wobei die Einrichtung (3a, 3b, 3c) dazu geeignet ist, unmittelbar vor der Öffnung des Magnetresonanztomographen (I) angeordnet zu werden,
- eine Lagerung (VI) zur beweglichen Lagerung der Einrichtung (3) und
- eine Steuereinheit (SE) zur Veränderung der Position des Leuchtmittels (II),

wobei die Steuereinheit (SE) elektrisch mit der Leiterschleife (III) und dem Leuchtmittel (II) verbunden ist und die erste Halterung (IV) derart mit der zweiten Halterung (V) mittels Kraftübertragung zusammenwirkt, dass die Position des Leuchtmittels (II) veränderbar ist, wobei das Magnetfeld des Magnetresonanztomographen (I) genutzt ist, um durch die bestromte Leiterschleife (III) die Position des Leuchtmittels (II) zu verändern.

6. Assistenzsystem (1) nach Anspruch 5, **dadurch gekennzeichnet dass** die erste Halterung (IV) in der Einrichtung (3a) zumindest bogenförmig ausgebildet ist und ein oder mehrere Leiterschleifen (III) mit Stromanschlüssen (VII) auf der ersten Halterung (IV) angeordnet sind, wobei die Leiterschleifen (III) separat mittels Strom ansteuerbar sind und im Streufeld des Magnetresonanztomographen (I) eine Kraft wirkt und die erste Halterung (IV) derart mit der zweiten Halterung (V) mittels Kraftübertragung zusammenwirkt, dass die Halterung (IV) um eine Längsachse L des Magnetresonanztomographen (I) rotierbar ist und die Koordinaten des Eintrittspunktes (IX) für das Instrument auf der Oberfläche des Patienten (VIII) markierbar sind.

7. Assistenzsystem (1) nach Anspruch 5, **dadurch gekennzeichnet, dass** die erste Halterung (IV) in der Einrichtung (3b) zumindest gerade ausgebildet ist und zumindest eine Leiterschleife (III) mit Stromanschlüssen (VII) auf der ersten Halterung (IV) angeordnet ist, wobei die Leiterschleife (III) mittels Strom ansteuerbar ist und eine Kraft wirkt und die erste Halterung (IV) derart mit der zweiten Halterung (V) mittels Kraftübertragung zusammenwirkt, dass die erste Halterung (IV) translatorisch oder linear in einer Translationsrichtung (T) bewegbar ist, derart, dass die zweite Halterung (V) in einer Rotationsrichtung (R) rotierbar ist und die Koordinaten des Eintrittspunktes (IX) für das Instrument auf der Oberfläche des Patien ten (VIII) markierbar sind.

8. Assistenzsystem nach einem der Ansprüche 5 - 7, **dadurch gekennzeichnet, dass** ein Seilzug vorgesehen ist, der mit der ersten Halterung (IV) und der zweiten Halterung (V) mittels Kraftübertragung derart zusammenwirkt, dass die Ausrichtung der zweiten Halterung (V) in einer Translationsrichtung (T) oder einer Rotationsrichtung (R) veränderbar ist und mittels der Leuchtmittel (II) Koordinaten des Eintrittspunktes (IX) auf der Oberfläche eines Patienten (VIII) markierbar sind.

9. Assistenzsystem (1) nach einem der vorhergehenden Ansprüche, **dadurch gekennzeichnet dass** die Leuchtmittel (II) ausgebildet sind als:

   - Diode und/oder
   - Laser und/oder
   - Spiegelsystem und/oder
   - Glühlampe mit Fokussierungseinrichtung

10. Verwendung eines operativen Assistenzsystems (1) für einen Magnetresonanztomographen (I) nach einem der vorhergehenden Ansprüche zur Anzeige eines Eintrittspunktes (IX) für ein Instrument auf der Oberfläche eines Patienten (VIII), wobei im Falle der Verwendung eines operativen Assistenzsystems nach einem der Ansprüche 1-4 oder 9, sofern auf einen der Ansprüche 1-4 rückbezogen, die drehbar gelagerte Einrichtung benachbart zu einer Mantelfläche (I$_M$) eines Magnetresonanztomographen (I) angeordnet ist, und im Falle der Verwendung eines operativen Assistenzsystems nach einem der Ansprüche 5-8 oder 9, sofern auf einen der Ansprüche 5-8 rückbezogen, die beweglich gelagerte Einrichtung unmittelbar vor der Öffnung des Magnetresonanztomographen (I) angeordnet ist.

**Claims**

1. An operative assistance system (1) for a magnetic resonance tomograph (I) for displaying an entry point (IX) for an instrument on the surface of a patient (VIII), at least comprising:

   - a rotatably mounted unit (3), at least comprising

     - a first holder (IV) accommodating at least one conductor loop (III) and

     - a second holder (V) with at least one light source (II) for displaying predefinable coordinates of the entry point (IX),
     - wherein the unit (3) is configured to be arranged adjacent to a lateral surface (I$_M$) of the magnetic resonance tomograph (I),
     - a mount (VI) for rotatably mounting the unit (3), and
     - a control unit (SE) for changing the position (alignment) of the light source (II),

   wherein the control unit (SE) is electrically connected to the conductor loop (III) and the light source (II) and the first holder (IV) interacts by means of force transmission with the second holder (V) such that the position of the light source (II) is changeable, wherein the magnetic field of the magnetic resonance tomograph (I) is used to change the position of the light source (II) by means of the energized conductor (III) loop.

2. The assistance system (1) as claimed in claim 1, **characterized in that** the assistance system (1) is configured to be arranged on an inner wall of the lateral surface (I$_M$) of the magnetic resonance tomograph (I).

3. The assistance system (1) as claimed in claim 1 or 2, comprising two conductor loops (III) accomodated by the first holder (IV), which are arranged perpendicularly to one another.

4. The assistance system (1) as claimed in any one of the preceding claims, **characterized in that** the unit (3) is mounted so it is rotatable in an angle φ on the mount (VI).

5. An operative assistance system (1) for a magnetic resonance tomograph (I) for displaying an entry point (IX) for an instrument on the surface of a patient (VIII), at least comprising:

   - a movably mounted unit (3a, 3b, 3c), at least comprising

     - a first holder (IV) accommodating at least one conductor loop (III) and

     - a second holder (V) with at least one light source (II) for marking predefinable coordinates of the entry point,
     - wherein the unit (3a, 3b, 3c) is configured to be arranged directly in front of the opening of the magnetic resonance tomograph (I),
     - a mount (VI) for movably mounting the unit, and
     - a control unit (SE) for changing the position of the light source (II),

   wherein the control unit (SE) is electrically connected to the conductor loop (III) and the light source (II) and the first holder (IV) interacts by means of force transmission with the second holder (V) such that the position of the light source (II) is changeable, wherein the magnetic field of the magnetic resonance tomograph (I) is used to change the position of the light source (II) by means of the energized conductor (III) loop.

6. The assistance system (1) as claimed in claim 5, **characterized in that** the first holder (IV) in the unit (3a) is formed at least curved and one or more conductor loops (III) having power connections (VII) are arranged on the first holder (IV), wherein the conductor loops (III) are separately drivable by means of current and a force acts in the stray field of the magnetic resonance tomograph (I) and the first holder (IV) interacts by means of force transmission with the second holder (V) such that the holder (IV) is rotatable about a longitudinal axis L of the magnetic resonance tomograph (I) and the coordinates of the entry point (IX) for the instrument are markable on the surface of the patient (VIII).

7. The assistance system (1) as claimed in claim 5, **characterized in that** the first holder (IV) in the unit (3b) is at least formed linear and at least one conductor loop (III) having power connections (VII) is arranged on the first holder (IV), wherein the conductor loop (III) is drivable by means of current and a force acts and the first holder (IV) interacts by means of force transmission with the second holder (V) such that the first holder (IV) is movable translationally or linearly in a translational direction T, such that the second holder (V) is rotatable in a rotational direction R and the coordinates of the entry point (IX) for the instrument are markable on the surface of the patient (VIII).

8. The assistance system as claimed in any one of claims 5 to 7, **characterized in that** a cable pull is provided, which interacts by means of force transmission with the first holder (IV) and the second holder (V) such that the alignment of the second holder (V) is changeable in a translational direction T or in a rotational direction R and coordinates of the entry point (IX) are markable on the surface of a patient (VIII) by means of the light source (II).

9. The assistance system (1) as claimed in any one of the preceding claims, **characterized in that** the light source (II) is formed as:

   - a diode and/or
   - a laser and/or
   - a mirror system and/or
   - an incandescent lamp having focusing unit.

10. Use of an operative assistance system (1) for a magnetic resonance tomograph (I) as claimed in any one of the preceding claims for displaying an entry point (IX) for an instrument on the surface of a patient (VIII), wherein in case of use of an operative assistance system according to one of the claims 1 to 4 or 9, if referred to one of the claims 1 to 4, the rotatably mounted unit is arranged adjacent to a lateral surface ($I_M$) of a magnetic resonance tomograph (I), and wherein in case of use of an operative assistance system according to one of the claims 5 to 8 or 9, if referred to one of the claims 5 to 8, the rotatably mounted unit is arranged directly in front of the opening of the magnetic resonance tomograph (I).

## Revendications

1. Système d'assistance chirurgical (1) pour un tomographe à résonance magnétique (I) permettant d'afficher un point d'entrée (IX) pour un instrument sur la surface d'un patient (VIII), comprenant au moins :

   - un dispositif (3) monté rotatif, comprenant au moins

      - une première attache (IV) recevant au moins une boucle conductrice (III), et

   - une deuxième attache (V) munie d'au moins un moyen luminescent (II) pour afficher des coordonnées prédéfinissables du point d'entrée (IX),
   - le dispositif (3) étant adapté pour être disposé à côté d'une surface latérale ($I_M$) du tomographe à résonance magnétique (I)
   - un dispositif formant palier (VI) pour le montage rotatif du dispositif (3), et
   - une unité de commande (SE) pour modifier la position du moyen luminescent (II),

   dans lequel l'unité de commande (SE) est reliée électriquement à la boucle conductrice (III) et au moyen luminescent (II) et la première attache (IV) coopère avec la deuxième attache (V) par transmission de force de telle sorte que la position du moyen luminescent (II) peut être modifiée, le champ magnétique du tomographe à résonance magnétique (I) étant utilisé pour modifier la position du moyen luminescent (II) par la boucle conductrice (III) mise sous tension.

2. Système d'assistance (1) selon la revendication 1, **caractérisé en ce que** le système d'assistance (1) est adapté pour être disposé sur une paroi intérieure de la surface latérale ($I_M$) du tomographe à résonance magnétique (I).

3. Système d'assistance (1) selon la revendication 1 ou 2, comprenant deux boucles conductrices (III) disposées sur la première attache (IV) qui sont disposées perpendiculairement l'une à l'autre.

4. Système d'assistance (1) selon l'une quelconque des revendications précédentes, **caractérisé en ce que** le dispositif (3) est monté rotatif sur le dispositif formant palier (VI) selon un angle φ.

5. Système d'assistance chirurgical (1) pour un tomographe à résonance magnétique (I) permettant d'afficher un point d'entrée (IX) pour un instrument sur la surface d'un patient (VIII), comprenant au moins :

- un dispositif (3a, 3b, 3c) monté mobile, comprenant au moins

- une première attache (IV) recevant au moins une boucle conductrice (III), et

- une deuxième attache (V) munie d'au moins un moyen luminescent (II) pour marquer des coordonnées prédéfinissables du point d'entrée (IX),
- le dispositif (3a, 3b, 3c) étant adapté pour être disposé directement devant l'ouverture du tomographe à résonance magnétique (I),
- un dispositif formant palier (VI) pour le montage mobile du dispositif (3), et
- une unité de commande (SE) pour modifier la position du moyen luminescent (II),

dans lequel l'unité de commande (SE) est reliée électriquement à la boucle conductrice (III) et au moyen luminescent (II) et la première attache (IV) coopère avec la deuxième attache (V) par transmission de force de telle sorte que la position du moyen luminescent (II) peut être modifiée, le champ magnétique du tomographe à résonance magnétique (I) étant utilisé pour modifier la position du moyen luminescent (II) par la boucle conductrice (III) mise sous tension.

6. Système d'assistance (1) selon la revendication 5, **caractérisé en ce que** la première attache dans le dispositif (3a) est réalisée au moins en forme d'arc, et une ou plusieurs boucles conductrices (III) avec des connecteurs électriques (VII) sont disposées sur la première attache (IV), les boucles conductrices (III) pouvant être excitées séparément au moyen d'un courant, et une force agissant dans le champ de dispersion du tomographe à résonance magnétique (I), et la première attache (IV) coopère avec la deuxième attache (V) par transmission de force de telle sorte que l'attache (IV) peut être amenée à tourner autour d'un axe longitudinal L du tomographe à résonance magnétique (I) et les coordonnées du point d'entrée (IX) pour l'instrument peuvent être marquées sur la surface du patient (VIII).

7. Système d'assistance (1) selon la revendication 5, **caractérisé en ce que** la première attache (IV) dans le dispositif (3b) est au moins rectiligne, et au moins une boucle conductrice (III) avec des connecteurs électriques (VII) est disposée sur la première attache (IV), dans lequel la boucle conductrice (III) peut être excitée par un courant et une force agit, et la première attache (IV) coopère avec la deuxième attache (V) par transmission de force de telle sorte que la première attache (IV) est mobile par translation ou de manière linéaire dans une direction de translation (T) de telle sorte que la deuxième attache (V) peut être amenée à tourner dans un sens de rotation (R) et les coordonnées du point d'entrée (IX) pour l'instrument peuvent être marquées sur la surface du patient (VIII).

8. Système d'assistance selon l'une quelconque des revendications 5 à 7, **caractérisé en ce qu'**un câble Bowden est prévu qui coopère avec la première attache (IV) et la deuxième attache (V) par transmission de force de telle sorte que l'alignement de la deuxième attache (V) dans une direction de translation (T) ou dans un sens de rotation (R) peut être modifié et que le moyen luminescent (II) permet de marquer les coordonnées du point d'entrée (IX) sur la surface d'un patient (VIII).

9. Système d'assistance (1) selon l'une quelconque des revendications précédentes, **caractérisé en ce que** les moyens luminescent (II) sont réalisés sous forme :

- de diode, et/ou
- de laser, et/ou
- de système de miroirs, et/ou
- de lampe à incandescence munie d'un dispositif de focalisation.

10. Utilisation d'un système d'assistance chirurgical (1) pour un tomographe à résonance magnétique (I) selon l'une quelconque des revendications précédentes permettant d'afficher un point d'entrée (IX) pour un instrument sur la surface d'un patient (VIII), dans laquelle, dans le cas de l'utilisation d'un système d'assistance chirurgical selon l'une quelconque des revendications 1 à 4 ou 9, lorsqu'elle dépend de l'une des revendications 1 à 4, le dispositif monté rotatif est disposé à côté d'une surface latérale ($I_M$) d'un tomographe à résonance magnétique (I), et dans le cas d'une utilisation d'un système d'assistance chirurgical selon l'une quelconque des revendications 5 à 8 ou 9, lorsqu'elle dépend de l'une des revendications 5 à 8, le dispositif monté mobile est disposé directement devant l'ouverture du tomographe à résonance magnétique (I) .

Fig. 1a

EP 3 452 843 B1

$$\vec{M} = \vec{M'} + \vec{M''}$$

Fig. 1b

Fig. 2

Fig. 3

EP 3 452 843 B1

Fig. 1'

Fig. 2'a

Fig. 2b

Fig. 3a

Fig. 3'b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011006650 A1
- DE 20000107 U1

- DE 102005046077 A1